# EUROPÄISCHE PATENTSCHRIFT

(11) **EP 2 044 009 B1**
(45) Veröffentlichungstag und Bekanntmachung des Hinweises auf die Patenterteilung: **25.02.2015**
(21) Anmeldenummer: 07787139.0
(22) Anmeldetag: 05.07.2007
(51) Int. Cl.: C07C 263/10, C07C 265/14

(54) **VERFAHREN ZUR HERSTELLUNG VON ISOCYANATEN**
METHOD FOR PRODUCING ISOCYANATES
PROCÉDÉ DE FABRICATION D'ISOCYANATES

(30) Priorität: 13.07.2006 EP 06117172
(43) Veröffentlichungstag der Anmeldung: 08.04.2009
(73) Patentinhaber: BASF SE, 67056 Ludwigshafen (DE)
(72) Erfinder: KNÖSCHE, Carsten, 67150 Niederkirchen (DE); STROEFER, Eckhard, 68163 Mannheim (DE); STÜTZER, Dieter, 67373 Dudenhofen (DE); SACHWEH, Bernd, 67149 Meckenheim (DE); LINSENBÜHLER, Markus, 67069 Ludwigshafen (DE); WÖLFERT, Andreas, 74906 Bad Rappenau (DE)
(86) Internationale Anmeldenummer: PCT/EP2007/056854
(87) Internationale Veröffentlichungsnummer: WO 2008/006775

(56) Entgegenhaltungen:
- EP-A- 1 275 639
- WO-A-03/045900

## Beschreibung

Gegenstand der Erfindung ist ein Verfahren zur Herstellung von Isocyanaten durch Umsetzung von Aminen mit Phosgen.

Polyisocyanate werden in großen Mengen hergestellt und dienen hauptsächlich als Ausgangsstoffe zur Herstellung von Polyurethanen. Ihre Herstellung erfolgt zumeist durch Umsetzung der entsprechenden Amine mit Phosgen.

Bei den aromatischen Isocyanaten haben Methylendi(phenylisocyanat) (MDI) und dessen höhere Homologen, und Toluylendiisocyanat (TDI), bei den aliphatischen Hexamethylendiisocyanat (HDI) und Isophorondiisocyanat (IPDI) die größte technische Bedeutung.

Die kontinuierliche Herstellung von organischen Isocyanaten durch Reaktion von primären organischen Aminen mit Phosgen ist vielfach beschrieben und wird im großtechnischen Maßstab durchgeführt (s. z. B. Ullmanns Enzyklopädie der Technischen Chemie, and 7 (Polyurethane), 3. neubearbeitete Auflage, Carl Hanser Verlag, München-Wien, S. 76ff (1993)).

In der Regel wird die Phosgenierung zweistufig durchgeführt. In der ersten Stufe der Phosgenierung wird das Amin mit Phosgen zu Carbamoylchlorid und Chlorwasserstoff und in einer parallelen Reaktion zu Aminhydrochlorid, welches im allgemeinen aufgrund seiner geringen Löslichkeit im Reaktionsgemisch als Feststoff ausfällt, umgesetzt. Die Reaktion zwischen Amin und Phosgen ist sehr schnell, stark exotherm und läuft schon bei niedrigen Temperaturen ab. Parallel dazu laufen weitere, die Ausbeute schmälernde, Reaktionen, wie die Bildung von Harnstoffen aus Isocyanat und Amin, ab. Um Nebenprodukte und Feststoffbildung zu minimieren, müssen daher Amin und Phosgen, gegebenenfalls in Mischung mit organischem Lösungsmittel, schnell vermischt und die Reaktion möglichst rückvermischungsfrei geführt werden. Daher erfolgt die erste Phosgenierstufe in der Regel in einem Mischorgan, vorzugsweise in einer Düse. Die zweite Stufe der Phosgenierung umfasst sowohl die Zersetzung des Carbamoylchlorids zum gewünschten Isocyanat und Chlorwasserstoff als auch die Phosgenierung des im wesentlichen als Feststoff vorliegenden Aminhydrochlorids zum Carbamoylchlorid. Die Temperatur der zweiten Phosgenierstufe ist in der Regel höher als die der ersten. Nachteilig an dieser Verfahrensführung ist es, dass die in der ersten Verfahrensstufe gebildeten Feststoffe aufgrund der gewählten Betriebsbedingungen als grobe, gegebenenfalls agglomerierte, und schwer zu phosgenierende Feststoffe ausfallen, die im weiteren Verlauf nicht vollständig umgesetzt werden können und neben Ausbeuteverlusten zu Verstopfungen und Fouling führen. Um dem entgegen zu wirken, müssen entweder die Temperatur, und damit der Druck, oder die Verweilzeit im Reaktor erhöht werden. Dies ist im allgemeinen aus sicherheitstechnischen, technischen und wirtschaftlichen Gründen nachteilig. Die Ausbeute der Reaktionsstufe wird wesentlich durch das Verhältnis aus Misch- und Reaktionsgeschwindigkeit bestimmt. Als vorteilhaft hat es sich erwiesen, als Mischorgan Düsen zu verwenden, wie beispielsweise in DE 100 26 142 A1 und EP 1 275 639 beschrieben. Haupthebel zur Beeinflussung der Mischgeschwindigkeit sind dabei die konstruktive Gestaltung der Düse und der realisierte Druckverlust über dieselbe. Von Nachteil ist hier, das mit steigendem phosgenseitigen Vordruck die technischen und sicherheitstechnischen Anforderungen an die Pumpensysteme steigen und insbesondere bei World-Scale-Anlagen erhebliche Anstrengungen zur Lösung der dabei auftretenden Probleme unternommen werden müssen. Um Nebenprodukte wie Harnstoffe und dessen Folgeprodukte zu minimieren, muss die gesamte Reaktion möglichst rückvermischungsfrei geführt werden. Reale Mischorgane und kontinuierlich betriebene Reaktoren besitzen einen von Null verschiedenen inhärenten Grad an Rückvermischung, der nicht unterschritten werden kann. Eine weitere Minimierung der durch Rückvermischung hervorgerufenen Nebenproduktbildung ist im allgemeinen nur noch durch eine diskontinuierliche Reaktionsführung möglich.

In jüngerer Zeit gewinnt die Herstellung von Isocyanaten in der Gasphase an Bedeutung. Bei diesen Verfahren liegt das Amin gasförmig vor und wird mit ebenfalls gasförmigen Phosgen umgesetzt. Dabei läuft die Reaktion üblicherweise oberhalb der Zersetzungstemperatur des Aminhydrochlorids ab. Damit wird das Ausfallen von Feststoffen in der Reaktionsstufe vermieden.

Derartige Verfahren sind bekannt und beispielsweise in EP 570 799, EP 593 334, WO 2004/026813 oder WO 03/045900 beschrieben.

Die Phosgenierung von Aminen in der Gasphase erlaubt eine erhebliche Reduzierung des im Verfahren umlaufenden Lösungsmittels, da prinzipiell auf dieses in der Reaktionsstufe verzichtet werden kann. Außerdem werden im allgemeinen höhere Ausbeuten als in der Flüssigphasenphosgenierung erreicht. Aufgrund der geringen Dichte der gasförmigen Edukte kann der Phosgen-Hold-Up erheblich reduziert werden, was deutliche sicherheitstechnische Vorteile bietet. Da bei der Gasphasenphosgenierung die Reaktionsbedingungen so gewählt werden, dass es zu keinem Ausfallen von Aminhydrochlorid kommen kann, werden die oben genannten Nachteile, die mit dem Anfall von schwer abreagierbaren Feststoffen verbunden sind, vermieden.

Mittels Gasphasenphosgenierung können nur solche Amine mit Phosgen umgesetzt werden, die sich mit vertretbarem technischen Aufwand in die Gasphase überführen lassen. Dies sind vorzugsweise die aliphatischen Diamine Hexamethylendiamin (HDA), Isophorondiamin (IPDA) sowie das aromatische Toluylendiamin (TDA). Die Herstellung von Methylendi(phenylisocyanat) (MDI), das technisch stets im Gemisch mit seinen höheren Homologen vorliegt, ist der Gasphasenphosgenierung nicht zugänglich, da das Zweikernprodukt nur mit hohem Aufwand und die höheren Homologen, das heißt Produkte mit drei oder mehr aromatischen Ringen, überhaupt nicht in die Gasphase überführt werden können.

Es war daher Aufgabe der vorliegenden Erfindung, ein Verfahren zur Herstellung von Isocyanaten durch Umsetzung von Aminen mit Phosgen zu entwickeln, welches
- eine Minimierung der Rückvermischung im Reaktor und der damit verbundenen Nebenproduktbildung sicherstellt,
- eine effektive Methode zur Vermischung von Amin und Phosgen bereitstellt, die geringe Mischzeiten bei gleichzeitig geringen phosgenseitigen Druckverlusten realisiert,
- sicherstellt, dass ausfallende Aminhydrochloride nicht zu schwer phosgenierbaren großen Aggregaten agglomerieren
und so eine hohe Raum-Zeit-Ausbeute und eine Steigerung der Qualität, insbesondere hinsichtlich der Reinheit, des NCO-Gehalts, der Molekulargewichtsverteilung und des Nebenproduktspektrums des Endprodukts realisiert.

Die Aufgabe wurde überraschenderweise dadurch gelöst, dass die Amine in Form eines Aerosols mit dem Phosgen umgesetzt werden.

Gegenstand der Erfindung ist demzufolge ein Verfahren zur Herstellung von Isocyanaten durch Umsetzung von Aminen mit Phosgen, dadurch gekennzeichnet, dass das Amin in Form eines Aerosols mit gasförmigem Phosgen umgesetzt wird.

Im folgenden wird unter der Tropfengrößenverteilung die volumengewichtete Größenverteilungsfunktion verstanden. Alle genannten Kenngrößen beziehen sich ebenfalls auf diese Verteilungsfunktion.

Das Aerosol sollte eine Tropfengrößenverteilung von 10 nm bis 1 mm, vorzugsweise von 100 nm bis 100 µm insbesondere von 0,2 bis 10 µm aufweisen. Die Tropfengrößenverteilung kann sehr breit oder sehr eng zwischen diesen Grenzen sein. Im idealen Fall ist die Tropfengrößenverteilung sehr eng. Als Maß für die Breite der Verteilung dient die auf den d50 der Tropfengrößenverteilung normierte Standardabweichung σ. Der d50 stellt die Tropfengröße dar, für die die Summenverteilungsfunktion den Wert 0,5 (50 %) erreicht. Für eine sehr breite Verteilung ist σ » 1. Für eine enge Verteilung gilt σ < 1 und für eine ideal monodisperse Verteilung der Wert σ = 0.

Allgemein gilt, dass die Größe der Tröpfchen so klein wie möglich sein sollte, da dies eine hohe Eindringgeschwindigkeit des Phosgens in die flüssige aminhaltige Phase gewährleistet. Desweiteren wird durch den realisierbaren Tropfendurchmesser die maximale Größe der ausfallenden Aminhydrochloridpartikel beschränkt. Deshalb gilt auch hier, dass ein feines gegenüber einem sehr groben Aerosol vorzuziehen ist. Allerdings muss darauf geachtet werden, dass das erzeugte Aerosol/Produkt auch von den nachgeschalteten Tropfen-/Staubabscheidern abgeschieden wird.

Die Tropfengrößenverteilung kann mittels gravimetrischer Messtechnik, wie beispielsweise Impaktor- oder Zyklonkaskadenmesstechnik ermittelt werden. Zudem können auch andere Verfahren wie beispielsweise laseroptische Verfahren, z. B. Laserbeugungssysteme, wie Welas Messsystem von Palas, Particle Doppler Annemometrie (PDA), Particle Image Velocimetry (PIV), oder auch Scanning Mobility Particle Sizer (SMPS-Systeme) zum Einsatz kommen. Einen Überblick über disperse Systeme und verschiedene Messverfahren gibt M. Stieß "Mechanische Verfahrenstechnik 1 ", Springer-Verlag, Berlin 1995, S. 4ff.

Die Erzeugung der Tropfen und damit des Aerosols kann über bekannte Aerosolerzeugungsverfahren, insbesondere mittels Düsen, erfolgen. In einer Ausführungsform sind dies Einstoff-Druckdüsen einschließlich Sonderbauformen, wie beispielsweise im Katalog Düsen-Schlick Produktübersicht: Vollkegeldüsen, Hohlkegeldüsen, Kreisl-Nebeldüsen, usw. In dieser Ausführungsform erfolgt die Zerstäubung über Druck.

In einer weiteren Ausführungsform kann eine Zweistoffdüse einschließlich Sonderbauformen, gegebenenfalls mit Zerstäubergas (Inerte oder auch Phosgen), eingesetzt werden (vgl. auch Katalog Düsen-Schlick Produktübersicht: Zweistoffdüsen, Mehrstoffdüsen). In dieser Ausführungsform erfolgt die Zerstäubung über ein zusätzliches Gas. Als Zerstäubergase können übliche Inertgase oder Phosgen eingesetzt werden.

In einer weiteren Ausführungsform kann eine Zerstäubung durch Ultraschall als Ultraschallverdüsung oder -vernebelung mittels einer Ultraschalldüse durchgeführt werden.

In einer weiteren Ausführungsform kann eine Zerstäubung durch ein rotierendes Rad, ein sogenanntes Zerstäuberrad, durchgeführt werden. Einen Überblick über verschiedensten Formen der Zerstäubung von Flüssigkeiten mit Düsen und anderen Zerstäubungsaggregaten gibt T. Richter in "Zerstäuben von Flüssigkeiten", Expert Verlag, Renningen 2004, S. 1ff. und G. Wozniak "Zerstäubungstechnik", Springer-Verlag, Berlin 2003, S. 57 - 88.

Die Amine können dabei als reine Substanz oder im Gemisch mit anderen Flüssigkeiten, die sich vor, während und nach der Umsetzung der Amine mit dem Phosgen inert verhalten, eingesetzt werden. Die Zerstäubung kann vorzugsweise bei einem Druck im Reaktionsraum im Bereich zwischen 1 und 20 bar (absolut), bevorzugt zwischen 1 - 10 bar, besonders bevorzugt zwischen 1 und 5 bar (absolut) erfolgen. Der Vordruck vor dem Zerstäubungsorgan hängt vom gewählten Verfahren, wie oben beschrieben, und der zu erzielenden Feinheit des Aerosols ab.

Die Zerstäubung der Amine wird bei der Verwendung der Amine als reine Substanz vorzugsweise bei einer Temperatur durchgeführt, die zwischen dem Schmelzpunkt und dem Siedepunkt des Amins liegt. Insbesondere wird die Zerstäubung bei der Temperatur durchgeführt, die der Anfangstemperatur, bei dem die nachfolgende Umsetzung des Amins mit dem Phosgen erfolgt, entspricht.

In einer bevorzugten Ausführungsform des erfindungsgemäßen Verfahrens wird das Amin oder das Amin-Lösungsmittelgemisch vor der Aerosolerzeugung unter Druck so überhitzt, dass während der Zerstäubung bei niedrigerem Druck ein Teil der Flüssigkeit schlagartig verdampft und so zu einer zusätzlichen Verfeinerung der entstehenden Tröpfchen führt. Dabei liegen die Vordrücke üblicherweise zwischen 1 und 20 bar, bevorzugt zwischen 1 und 10 bar Absolutdruck über dem Reaktordruck. Der Vordruck, d. h. der Überhitzungsgrad vor dem Zerstäubungsorgan, hängt, wie oben beschrieben, von der gewünschten Tropfengrößenverteilung und dem gewählten Zerstäubungsverfahren ab. Bei dieser Ausführungsform des Verfahrens wird durch Erhöhung der Temperatur und des Drucks vor dem und einer schlagartige Entspannung des zu zerstäubenden Gemischs nach dem Zerstäubungsorgan eine Verringerung der Tröpfchengröße und eine Verschiebung der Tröpfchengrößenverteilung hin zu kleineren Tröpfchen bewirkt.

Eine weitere Möglichkeit zur Verringerung der Tröpfchengröße besteht im Anlegen eines elektrischen Feldes zwischen dem Zerstäuber, in diesem Falle vorzugsweise einer Düse, und einer Gegenelektrode, dem sogenannten Elektrosprayverfahren. Die auftretenden elektrischen Kräfte führen zusätzlich zu einer Verringerung der Tropfengröße des erzeugten Aerosols. Genauere Erläuterungen zur Wirkungsweise und zu Ausführungsformen finden sich z. B. in H. Wiggers, P. Walzel, "Elektrostatisches Zerstäuben von Flüssigkeiten", Chem. Ing. Tech. 69 (1997) 1066 - 1073; A. G. Bailey: Electrostatic Spraying of Liquids, Res. Stud. Press Ltd Taunton, Somerset 1988; D. Michelson: Electrostatic Atomization, Adam Hilger, Bristol-New York 1990.

Auch durch die oben beschriebene Zugabe von Flüssigkeiten, die sich vor, während und nach der Umsetzung der Amine mit dem Phosgen inert verhalten, kann eine Verringerung Tröpfchengröße erreicht werden. Einerseits lassen sich auf diese Weise wichtige, die Tropfengröße beeinflussende, physikalische Eigenschaften des aminhaltigen Flüssigkeitsstroms, wie dessen Viskosität oder Oberflächenspannung, gezielt beeinflussen. Andererseits kann eine zusätzliche Tropfengrößenzerkleinerung durch verdampfendes Lösungsmittel in Kombination mit dem oben beschriebenen Verfahren der entspannenden Zerstäubung eines überhitzten Flüssigkeitsstroms erreicht werden. Als inerte Verbindungen können vorzugsweise organische Lösungsmittel eingesetzt werden. Insbesondere kommen aromatische Lösungsmittel, die auch halogeniert sein können zum Einsatz. Beispiele hierfür sind Toluol, Monochlorbenzol, o- oder p-Dichlorbenzol, Trichlorbenzol, Chlortoluole, Chlorxylole, Chlorethylbenzol, Chlornaphtaline, Chlordiphenyle, Xylol, Dekahydronaphtalin, Benzol und andere Gemische. Weitere Beispiele für organische Lösungsmittel sind Methylenchlorid, Perchlorethylen, Hexan, Diethylisophthalat, Tetrahydrofuran (THF), Dioxan, Trichlorfluormethan, Butylacetat und Dimethylformamid (DMF).

Eine weitere Möglichkeit, die Tröpfchengröße zu optimieren, besteht im Einsatz von Klassierverfahren. Dadurch können zu große Tropfen vor der Reaktion mit dem Phosgen abgeschieden werden. Dies kann beispielsweise durch den Einbau von Lochblenden oder Fritten zwischen den Aerosolgenerator und die Reaktionszone erfolgen. Es können auch andere Verfahren zur Klassierung eingesetzt werden, beispielsweise Zentrifugalabscheider, Schwerkraftabscheider oder Elektrofilter.

Der abgetrennte Flüssigkeitsstrom wird vorzugsweise rezirkuliert, das heißt zum Ausgangsprodukt vor der Aerosolbildung zurückgeführt.

Um eine Kontamination des rückgeführten beziehungsweise rezirkulierten Amins mit Phosgen zu vermeiden und eine Reaktion dieses Flüssigkeitsanteils außerhalb des Reaktors zu verhindern, kann der Bereich der Tropfenklassierung mit Inertgas im Überdruck, wie beispielsweise Stickstoff, beaufschlagt werden. Dadurch wird ein Eindringen von Reaktionsgas, wie beispielsweise Phosgen, in den Klassierraum verhindert.

Die Umsetzung der als Aerosol vorliegenden Amine mit Phosgen erfolgt üblicherweise bei Drücken von 1 - 20 bar (absolut), bevorzugt 1 - 10 bar (absolut), besonders bevorzugt 1 - 5 bar (absolut) und Temperaturen von 50 - 350 °C, bevorzugt 50 - 250 °C, besonders bevorzugt 90 - 150 °C. Dabei wird Phosgen dem Reaktor so zugemischt, dass innerhalb der Tröpfchen ein molarer Überschuss gegenüber den Amingruppen von 1:1 bis 20:1, bevorzugt 1:1 bis 10:1, besonders bevorzugt 1:1 bis 5:1 realisiert wird. Die Reaktion kann in Rohrreaktoren, Sprühtürmen oder auch Schlaufenreaktoren durchgeführt werden. Prinzipiell lassen sich aber auch andere Bauformen, die hier nicht exemplarisch aufgeführt wurden, nutzen. Erfolgt die Aerosolbildung nicht über eine mit Phosgen betriebene Zweistoffdüse, muss nach der Versprühung des Amins bzw. des Amin-Lösungsmittelgemischs dem Aerosol gasförmiges Phosgen so beigemischt werden, das eine möglichst gleichmäßige Vermischung der beiden Eduktströme erfolgt. Dabei ist es besonders wichtig, die Zeit bis zur Erreichung der Homogenität möglichst gering zu halten. Um dies zu gewährleisten können, alle dem Fachmann geläufigen technischen Methoden, wie die verteilte Zuführung des Phosgens, im Gleichstrom oder im Gegenstrom, die zentrale, axiale verdrallte Phosgeneinspeisung oder die Vermischung der Eduktströme in einer oder mehreren Düsen, wie Ringspaltdüsen oder Gegenstromdüsen, angewandt werden. Vorteilhaft kann es auch sein, das Phosgen mittels langsam verdampfender und Phosgen enthaltenden Lösungsmitteltropfen in das Aminaerosol einzumischen.

Die Reaktion kann bis zum vollständigen Umsatz zum Isocyanat innerhalb des Aersolreaktors geführt werden. Es kann aber auch vorteilhaft oder notwendig sein, einen Teilumsatz, insbesondere von Resten Aminhydrochlorid, in einem Nachreaktor in flüssiger Phase durchzuführen. Bei dem Nachreaktor kann es sich um übliche Reaktorbauformen unterschiedlichen Rückvermischungsgrades, wie Rührkesseln, Schlaufenreaktoren oder Rohrreaktoren handeln. Die Abscheidung des vollständig oder nur teilweise umgesetzten Aerosols erfolgt über bekannte Tropfen- bzw. Partikelabscheideverfahren wie beispielsweise Filter, Demister, Zentrifugalabscheider, Lamellenabscheider oder Schwerkraftabscheider. Eine Beschreibung von verschiedenen Tropfenabscheidern kann A. Bürkholz "Droplet Separation", VCH Verlagsgesellschaft, Weinheim 1989, S. 17ff. entnommen werden. F. Löffler "Staubabscheiden", Georg Thieme Verlag, Stuttgart 1988, S. 32ff. und M. Stieß "Mechanische Verfahrenstechnik 2", Springer-Verlag, Berlin 1997, S. 1-53 geben einen Überblick über Bauformen von Partikelabscheidern.

Nach der Reaktion wird das bei der Umsetzung entstehende Stoffgemisch üblicherweise in Isocyanat(e), inerte Verbindungen, vorzugsweise Lösungsmittel, nicht umgesetztes Phosgen und Chlorwasserstoff aufgetrennt. Geringe Mengen von Nebenprodukten, die im Isocyanat verbleiben, können mittels zusätzlicher Rektifikation oder auch Kristallisation vom erwünschten Isocyanat getrennt werden.

Das nicht umgesetzte Phosgen wird zumeist, gegebenenfalls nach einer Reinigung, zurückgeführt und wieder zur Phosgenierung eingesetzt. Der bei der Reaktion entstandene Chlorwasserstoff kann, gegebenenfalls nach einer Reinigung, zur Herstellung von Vinylchlorid oder zur Herstellung von Salzsäure eingesetzt werden. Es ist auch möglich, den Chlorwasserstoff nach dem sogenannten Deacon-Verfahren mit Sauerstoff zu Chlor umzusetzen und dieses in die Phosgensynthese zu rezyklieren.

Als Amine können alle zur Herstellung von Isocyanaten üblichen Amine eingesetzt werden. Dies sind beispielsweise, wie oben beschrieben, aliphatische Diamine wie Hexamethylendiamin (HDA), Isophorondiamin (IPDA) sowie das aromatische Toluylendiamin (TDA) sowie Methylendi(phenyldiamin) (MDA) im Gemisch mit seinen höheren Homologen. Insbesondere bei der Phosgenierung von MDA kann das erfindungsgemäße Verfahren besonders vorteilhaft eingesetzt werden.

Gegenüber der Flüssigphasenphosgenierung weist das hier vorgeschlagene Verfahren folgende wesentliche Vorteile auf: Die Rückvermischung von Reaktionsprodukt in den noch nicht abreagierten Eduktstrom wird aufgrund der räumlichen Begrenztheit der Flüssigkeitströpfchen minimiert. Durch Variation der Tropfengröße lassen sich sehr kurze Mischzeiten zwischen Phosgen und Amin realisieren und steuern. Dabei können die Druckverluste phosgenseitig gering gehalten werden, ohne das Mischergebnis wesentlich zu beeinflussen. Die kurzen Mischzeiten und der geringe Rückvermischungsgrad führen zu minimalen Ausbeuteverlusten und hohen Produktqualitäten, d. h. guten Farbzahlen, geringen Chlorgehalten, hohen NCO-Zahlen, einer optimalen Molekulargewichtsverteilung etc. Die Partikelgröße der ausfallenden Aminhydrochloride wird aufgrund der begrenzenden Tropfengröße kleiner einem Maximalwert gehalten (Ausschluss weiterer Agglomeration). Dies stellt minimale Verweilzeiten für die Aminhydrochloridphosgenierung und eine geringere Foulinganfälligkeit des Verfahrens sicher. Im Gegensatz zur Gasphasenphosgenierung lassen sich mittels Aerosolphosgenierung auch Amine oder Amingemische phosgenieren, die hohe Siedetemperaturen aufweisen und deshalb gar nicht oder nur mit hohem Aufwand in die Gasphase überführt werden können. Des weiteren ist es möglich, auch unterhalb des Siedpunktes des Amins die Reaktion zu führen. Thermische induzierte Qualitäts- oder Ausbeuteverluste können dadurch vermieden werden. Außerdem kann auf die energieintensive Verdampfung des Amins oder des Amingemischs bei hohen Temperaturen verzichtet werden.

Die Erfindung soll an dem nachfolgenden Beispiel näher beschrieben werden.

### Beispiel:

MDA aus der sauer katalysierten Umsetzung von Anilin mit Formaldehyd wurde mit einer Zweistoffdüse (Schlick Modellreihe 970) in einem Sprüh-Rohrreaktor bei 10 bar Druck verdüst. Es wurde ein MDA Volumenstrom von 0,36 l/h (0,39 kg/h) in Mischung mit einem MCB Volumenstrom von 1,23 l/h (0,9 kg/h) pro Düse realisiert (1,6 l/h Fluid bei 50 °C). Zur Zerstäubung lag ein Überdrück an der Düse an, so dass das MDA überhitzt war, und es kam Stickstoff im Überdruck als Zerstäubungsgas mit zum Einsatz. Overspray wurde durch ein Lochblech zurückgehalten und rezirkuliert, so dass das MDA in Form von Tropfen mit einem Durchmesser von <10µm in den Reaktor dosiert wurde. Das so erzeugte Aerosol wurde mit gasförmig eingedüstem Phosgen im Massenverhältnis 1:7,5 umgesetzt (2,88 kg/h bei 90 °C). Das entstehende MDI wurde mittels eines Zentrifugaltropfenabscheiders abgeschieden, aus dem Prozess ausgeschleust und aufgearbeitet.

Das überschüssige Phosgen und der entstehende Chlorwasserstoff wurden aus dem Reaktor ausgeschleust und aufgetrennt. Das Phosgen wurde in das Verfahren zurückgeführt.

## Patentansprüche

1. Verfahren zur Herstellung von Isocyanaten durch Umsetzung von Aminen mit Phosgen, **dadurch gekennzeichnet, dass** das Amin oder ein Gemisch aus Amin und einem Lösungsmittel in Form eines Aerosols mit gasförmigem Phosgen vermischt und anschließend das Amin mit Phosgen umgesetzt wird, wobei das Aerosol eine Tropfengrößenverteilung von 10 nm bis 1 mm aufweist.

2. Verfahren nach Anspruch 1, **dadurch gekennzeichnet, dass** das Aerosol mittels Düsen erzeugt wird.

3. Verfahren nach Anspruch 1, **dadurch gekennzeichnet, dass** das Aerosol mittels Einstoff-Druckdüsen erzeugt wird.

4. Verfahren nach Anspruch 1, **dadurch gekennzeichnet, dass** das Aerosol mittels Zweistoffdüsen erzeugt wird.

5. Verfahren nach Anspruch 1, **dadurch gekennzeichnet, dass** das Aerosol mittels Zerstäuberrad erzeugt wird.

6. Verfahren nach Anspruch 1, **dadurch gekennzeichnet, dass** das Aerosol mittels Ultraschalldüse erzeugt wird.

7. Verfahren nach Anspruch 1, **dadurch gekennzeichnet, dass** das Aerosol mittels Elektrosprayverfahren erzeugt wird.

8. Verfahren nach Anspruch 4, **dadurch gekennzeichnet, dass** die Zerstäubung über ein zusätzliches Gas erfolgt.

9. Verfahren nach Anspruch 8, **dadurch gekennzeichnet, dass** als zusätzliches Gas Inertgase oder Phosgen eingesetzt werden.

10. Verfahren nach Anspruch 1, **dadurch gekennzeichnet, dass** als Amine Hexamethylendiamin (HDA), Isophorondiamin (IPDA) sowie das aromatische Toluylendiamin (TDA) sowie Methylendi(phenyldiamin) (MDA) im Gemisch mit seinen höheren Homologen eingesetzt werden.

11. Verfahren nach Anspruch 1, **dadurch gekennzeichnet, dass** die Amine als reine Substanz eingesetzt werden.

12. Verfahren nach Anspruch 1, **dadurch gekennzeichnet, dass** die Amine gemeinsam mit Verbindungen, die sich während und nach der Umsetzung mit dem Phosgen inert verhalten, eingesetzt werden.

13. Verfahren nach Anspruch 1, **dadurch gekennzeichnet, dass** die Zerstäubung bei einem Differenzdruck zwischen 1 und 20 bar absolut durchgeführt wird.

14. Verfahren nach Anspruch 1, **dadurch gekennzeichnet, dass** die Umsetzung der Amine mit Phosgen bei Drücken von 1 - 20 bar (absolut) erfolgt.

15. Verfahren nach Anspruch 1, **dadurch gekennzeichnet, dass** die Umsetzung der Amine mit Phosgen bei Temperaturen von 50 - 350 °C erfolgt.

16. Verfahren nach Anspruch 1, **dadurch gekennzeichnet, dass** die Umsetzung der Amine mit Phosgen in Rohrreaktoren, Sprühtürmen oder Schlaufenreaktoren durchgeführt wird.

## Claims

1. A process for preparing isocyanates by reacting amines with phosgene, wherein the amine or a mixture of amine and a solvent is mixed in the form of an aerosol with gaseous phosgene and the amine is subsequently reacted with phosgene, where the aerosol has a droplet size distribution of from 10 nm to 1 mm.

2. The process according to claim 1, wherein the aerosol is produced by means of nozzles.

3. The process according to claim 1, wherein the aerosol is produced by means of single-fluid pressure nozzles.

4. The process according to claim 1, wherein the aerosol is produced by means of two-fluid nozzles.

5. The process according to claim 1, wherein the aerosol is produced by means of a rotary atomizer disk.

6. The process according to claim 1, wherein the aerosol is produced by means of an ultrasonic nozzle.

7. The process according to claim 1, wherein the aerosol is produced by means of the electrospraying method.

8. The process according to claim 4, wherein the atomization is effected by means of an additional gas.

9. The process according to claim 8, wherein inert gases or phosgene are used as additional gas.

10. The process according to claim 1, wherein hexamethylenediamine (HDA), isophoronediamine (IPDA) and the aromatic toluenediamine (TDA) and methylenedi(phenylamine) (MDA) in admixture with its higher homologues are used as amines.

11. The process according to claim 1, wherein the amines are used as pure substances.

12. The process according to claim 1, wherein the amines are used together with compounds which display inert behavior during and after the reaction with the phosgene.

13. The process according to claim 1, wherein atomization is carried out at a differential pressure in the range from 1 to 20 bar absolute.

14. The process according to claim 1, wherein the reaction of the amines with phosgene is carried out at pressures of 1 - 20 bar (absolute).

15. The process according to claim 1, wherein the reaction of the amines with phosgene is carried out at temperatures of 50 - 350°C.

16. The process according to claim 1, wherein the reaction of the amines with phosgene is carried out in tube reactors, spray towers or loop reactors.

## Revendications

1. Procédé de fabrication d'isocyanates par mise en réaction d'amines avec du phosgène, **caractérisé en ce que** l'amine ou un mélange d'amine et d'un solvant sous la forme d'un aérosol est mélangé avec du phosgène gazeux, puis l'amine est mise en réaction avec le phosgène, l'aérosol présentant une distribution des tailles de gouttes de 10 nm à 1 mm.

2. Procédé selon la revendication 1, **caractérisé en ce que** l'aérosol est généré par des buses.

3. Procédé selon la revendication 1, **caractérisé en ce que** l'aérosol est généré par des buses sous pression à un constituant.

4. Procédé selon la revendication 1, **caractérisé en ce que** l'aérosol est généré par des buses à deux constituants.

5. Procédé selon la revendication 1, **caractérisé en ce que** l'aérosol est généré par une roue d'atomiseur.

6. Procédé selon la revendication 1, **caractérisé en ce que** l'aérosol est généré par une buse à ultrason.

7. Procédé selon la revendication 1, **caractérisé en ce que** l'aérosol est généré par un procédé à électronébulisation.

8. Procédé selon la revendication 4, **caractérisé en ce que** l'atomisation a lieu par un gaz supplémentaire.

9. Procédé selon la revendication 8, **caractérisé en ce que** des gaz inertes ou du phosgène sont utilisés en tant que gaz supplémentaire.

10. Procédé selon la revendication 1, **caractérisé en ce que** l'hexaméthylène-diamine (HDA), l'isophoronediamine (IPDA), ainsi que la toluylène-diamine aromatique (TDA), ainsi que la méthylènedi(phényldiamine) (MDA) en mélange avec ses homologues supérieurs sont utilisées en tant qu'amines.

11. Procédé selon la revendication 1, **caractérisé en ce que** les amines sont utilisées sous la forme d'une substance pure.

12. Procédé selon la revendication 1, **caractérisé en ce que** les amines sont utilisées conjointement avec des composés qui se comportent de manière inerte pendant et après la réaction avec le phosgène.

13. Procédé selon la revendication 1, **caractérisé en ce que** l'atomisation est réalisée à une pression différentielle comprise entre 1 et 20 bar absolu.

14. Procédé selon la revendication 1, **caractérisé en ce que** la réaction des amines avec le phosgène a lieu à des pressions de 1 à 20 bar (absolu).

15. Procédé selon la revendication 1, **caractérisé en ce que** la réaction des amines avec le phosgène a lieu à des températures de 50 à 350 °C.

16. Procédé selon la revendication 1, **caractérisé en ce que** la réaction des amines avec le phosgène est réalisée dans des réacteurs tubulaires, des tours de pulvérisation ou des réacteurs à boucle.
